# EUROPÄISCHE PATENTSCHRIFT

(11) **EP 1 935 342 B1**
(45) Veröffentlichungstag und Bekanntmachung des Hinweises auf die Patenterteilung: **25.02.2015**
(21) Anmeldenummer: 06026291.2
(22) Anmeldetag: 19.12.2006
(51) Int. Cl.: A61B 6/04, A61B 6/12, A61G 13/12

(54) **Artefakt-Eliminierung für eine medizintechnische Beckenregistrierung mit getrackter, systembekannter Beckenstütze**
Artefact elimination for medical technology pelvic registration with tracked pelvic support known to the system
Elimination d'objets pour un enregistrement technique médical du bassin à l'aide d'un soutien de bassin suivi connu du système

(43) Veröffentlichungstag der Anmeldung: 25.06.2008
(73) Patentinhaber: Brainlab AG, 85622 Feldkirchen (DE)
(72) Erfinder: Wagner, Benjamin, 80469 München (DE); Neubauer, Timo, 85586 Poing (DE)
(74) Vertreter: Schwabe - Sandmair - Marx

(56) Entgegenhaltungen:
- EP-A- 1 522 300
- EP-A- 1 627 601
- WO-A-2004/017263
- WO-A-2005/084541
- WO-A2-2005/009220
- US-A- 5 442 674
- US-A1- 2002 154 735
- US-A1- 2004 199 072
- US-B1- 6 493 573

## Beschreibung

Die Erfindung betrifft das technische Gebiet der Artefakt-Eliminierung für eine medizinische Beckenregistrierung mit getrackter Beckenstütze.

Bei Beckenoperationen, insbesondere bei Hüftersatz-Operationen, werden Patienten meist auf der Seite liegend operiert. Dabei ist es notwendig, das Becken des Patienten zu fixieren. Dies geschieht über Patientenpositionierer, die Stützvorrichtungen für den Unterkörperbereich des Patienten aufweisen. In dieser Lage ist es schwierig, Navigationsinformationen durch das Abgreifen von Körper-Landmarken (ASIS ("anterior superior ilicic spin")- und Pubis-Punkte) im Rahmen einer medizinischen Navigation zu ermitteln, weil diese charakteristischen Becken-Landmarken zumindest im unteren Bereich meist kaum zugänglich sind. Deshalb wird oft mit einer Röntgen- oder Fluoroskopie-Registrierung gearbeitet.

Viele der herkömmlichen Patientenpositionierer verwenden Kissen oder Stäbe, die an einem Armsystem des Operationstisches oder an ähnlichen festen Einrichtungen fixiert sind. Ein solcher mechanischer Positionierer ist beispielsweise aus der US 6,311,349 bekannt.

Neben den mechanischen Positionieren gibt es noch diejenigen, die Navigations-Referenzeinrichtungen für eine medizintechnische Navigation aufweisen, und ein solcher ist beispielsweise aus der WO 2004/089 192 A2 bekannt. Die Referenzeinrichtungen werden verwendet, um die ASIS- und Pubis-Punkte indirekt zu lokalisieren und damit die vordere Beckenebene zu registrieren.

US 2004/0199072 A1 offenbart eine Vorrichtung und ein Verfahren zum Positionieren eines Patienten, die bzw. das geeignet ist, den Einfluss einer Patientenkopfstütze auf ein zur Positionierung des Patienten verwendetes, von Spulen erzeugtes elektromagnetisches Feld zu eliminieren.

EP 1 627 601 offenbart ein Verfahren zum Entfernen von Artefakten, die ein metallisches Implantat in einer Röntgen oder Fluoroskopieabbildung verursacht. Dabei wird ein CAD-Modell des Implantats mit der Position des Implantats aus der Fluoroskopieabbildung verrechnet. Die Position, an der das Modell subtrahiert werden muss, wird mittels eines Pointers bestimmt, der das Implantat in der Fluoroskopieabbildung abtastet.

US 6,493,573 B1 offenbart ein Verfahren, das zum Einsatz mit der Vorrichtung bzw. dem Verfahren aus US 2004/0199072 A1 geeignet ist und die Berechnung von Korrekturen für das elektromagnetische Feld erlaubt.

US 2002/0154735 A1 offenbart ein Verfahren zum Entfernen von Artefakten aus Röntgenbildem, bei dem von Markervorrichtungen im Bild verursachte Artefakte entfernt werden, indem ein bekanntes Intensitätsbild des Markers von dem aufgenommenen Bild abgezogen wird.

WO 2005/009220 A2 offenbart ein Verfahren zum Registrieren von Ultraschall- und C-Bogen-Fluoroskopiebildern, bei dem unerwünschterweise mitabgebildete Marker aus einem Röntgenbild basierend auf ihrer bekannten Gestalt, Orientierung und geschätzte relativen Position sowie der daraus ermittelten dreidimensionalen Lage durch Entfernen ihrer Umrisse eliminiert werden.

Ein Hauptproblem der existierenden Positionierungseinrichtungen zeigt sich, wenn Fluoroskopiebilder aufgenommen werden. Es liegt in der Natur der Positionierungseinrichtungen, dass mindestens ein Teil ihrer Stützvorrichtungen im Strahlengang der Fluoroskopieaufnahme liegt und deshalb Artefakte auf der Aufnahme entstehen. Auch wenn die gewählten Materialien für die Stützvorrichtung für Röntgenstrahlung durchlässig sind, entstehen noch immer Schatten und Kanten auf den Bildern. Diese Artefakt-Konturen überlagern die anatomische Struktur (Becken) und könnten in einer Missinterpretation der Aufnahmen resultieren. Die Navigationssoftware erfasst anatomische Strukturen, um das Becken zu registrieren, und die angesprochenen Schatten und Kanten könnten hier zu einer falschen Konturenwahl und somit zu einer falschen Registrierung führen. Zusätzlich wird es schwierig, Punkte in dem Bereich der Stützen-Abbildungen zu definieren, weil dort aufgrund der Artefakte der Kontrast leidet.

Es ist die Aufgabe der vorliegenden Erfindung, eine Artefakt-Eliminierung bei der medizinischen Beckenregistrierung vorzuschlagen, welche die oben genannten Nachteile des Standes der Technik überwindet. Insbesondere sollen Fehlerregistrierungen vermieden werden.

Diese Aufgabe wird gemäß einem ersten Aspekt der Erfindung durch ein Artefakt-Eliminierungsverfahren gemäß dem Anspruch 1 und gemäß einem zweiten Aspekt der Erfindung durch ein Artefakt-Eliminierungssystem gemäß dem Anspruch 8 gelöst. Die Unteransprüche definieren bevorzugte Ausführungsformen der Erfindung.

Das erfindungsgemäße Artefakt-Eliminierungsverfahren dient der medizintechnischen Beckenregistrierung zur positionellen Registrierung eines mit mindestens einer Stütze abgestützten Patientenbeckens, und es weist die folgenden Schritte auf:
- die geometrischen Daten der Stütze sowie eine vorbekannte positionnelle Zuordnung zwischen der Stütze und einer Navigations-Referenzeinrichtung werden im Datenspeicher eines für die Registrierung verwendeten medizintechnischen Navigationssystems abgespeichert,
- mindestens zwei Röntgen- bzw. Fluoroskopieaufnahmen des Patientenbeckens werden erstellt, wobei die Stütze sich im Strahlengang der Aufnahme befindet,
- die Position der Stütze und des Röntgen- bzw. Fluoroskopiegeräts während der Aufnahme werden durch das Navigationssystem basierend auf der Erfassung der der Stütze positionell zugeorneten Navigations-Referenzeinrichtung ermittelt,
- mit Hilfe der ermittelten Positionsdaten für die Stütze und das Röntgen- bzw. Fluoroskopiegerät wird die Abbildung der Stütze in der Aufnahme identifiziert,
- die Aufnahme wird für die Registrierung verwendet, wobei die Abbildungselemente der identifizierten Abbildung der Stütze als bei der Registrierung nicht zu verwendende Teile der Abbildung definiert werden.

Mit anderen Worten nutzt die vorliegende Erfindung die Kenntnis über die Dimensionen bzw. Abmessungen der Stütze sowie die Kenntnis über die Position der Stütze dazu, die Stützenabbildung bei der Registrierung unberücksichtigt zu lassen. Weil man weiß, wo die Stütze liegt und wie ihre Abbildung aussehen wird, kann man vermeiden, dass die abgebildeten Stützenelemente fälschlicherweise der Registrierung zugrunde gelegt werden.

Durch die Erfindung wird es möglich, einen Patienten seitlich während einer Beckenoperation zu positionieren und problemlos die anatomischen Beckenlandmarken zu registrieren, wenn die Prozedur durch eine medizintechnische Navigation unterstützt wird. Das Problem mit Stützen-Artefakten in der Aufnahme tritt nicht mehr auf, weil die Stütze anhand der Referenzeinrichtung getrackt werden kann, ihre geometrischen Daten (Abmessungen, Innenstruktur, etc.) in einer Datenbank der Navigationssoftware hinterlegt werden und die so geschaffene Erkennbarkeit der Stütze dann verwendet wird, um im Bild die Stützen-Artefakte zu berechnen, um sie bei der Registrierung unberücksichtigt zu lassen.

Bei einer Ausführungsform der Erfindung werden die Abbildungselemente der identifizierten Abbildung der Stütze von der Aufnahme subtrahiert, was durch geeignete Grafiksoftware und Grafikhardware geschehen kann. Im Bild verbleiben die tatsächlich zu verwendenden Beckenpunkte und Beckenkonturen für die Registrierung.

Grundsätzlich können alle Stützenelemente, die in der Aufnahme abgebildet werden, aus der Aufnahme "herausgerechnet" werden. Andererseits genügt es oft, die abgebildeten Konturen der Stütze in der Aufnahme zu identifizieren und als nicht zu verwendende Teile zu definieren.

Bei der Identifizierung der Stützenabbildung besteht die Möglichkeit, die Grauwerte der Stützen-Abbildungselemente als Identifizierungshilfsmittel zu verwenden, insbesondere bekannte oder im wesentlichen bekannte Grauwerte, wie sie von den Materialien der Stütze üblicherweise erzeugt werden. Bei der Identifizierung der Stützenabbildung ist es von Vorteil, wenn Abstandsverhältnisse der Stützenelemente (Außenkanten, Innenkanten, Ecken) verwendet werden, d.h. bekannte Abstände solcher Elemente zu bestimmten Punkten oder Teilen der Stütze, die sich gut in der Aufnahme identifizieren lassen. Solche Ausgangspunkte für die Berechnung der Stützenabbildung können beispielsweise deutlich abbildbare Stützenelemente sein, es besteht aber auch die Möglichkeit, zusätzliche Marker in oder an der Stütze anzubringen und diese als Identifizierungshilfsmittel bzw. Ausgangspunkte für Abstandsbestimmungen zu benutzen. Bei einer Ausführungsform der Erfindung können auch gedachte Linien oder Ebenen als solche Ausgangspunkte oder Identifizierungshilfsmittel verwendet werden, beispielsweise eine gedachte Verbindungslinie zwischen speziellen, deutlich abbildbaren Markern.

Das erfindungsgemäße Artefakt-Eliminierungssystem dient der Eliminierung von Artefakten in Röntgen- bzw. Fluoroskopieaufnahmen, die bei einem medizinischen Beckenregistrierungsverfahren verwendet werden, das zur positionellen Registrierung eines mit mindestens einer Stütze abgestützten Patientenbeckens dient. Das System umfasst:
- ein Röntgen- bzw. Fluoroskopiegerät;
- ein medizintechnisches Navigationssystem, das eingerichtet ist, Positionsdaten des Röntgen- bzw. Fluoroskopiegeräts zu bestimmen;
- eine Trackingvorrichtung, welche die Position einer Navigations-Referenzeinrichtungen bestimmen kann;
- eine der Stütze in vorbekannter Weise positionell zugeordneten Navigations-Referenzeinrichtung;
- einen Datenspeicher im Navigationssystem, in dem die geometrischen Daten der Stütze und die vorbekannten positionellen Zuordnung zwischen Stütze und der Navigations-Referenzeinrichtung abgespeichert werden; und
- eine Logik im Navigationssystem, welche eingerichtet ist, aufgrund der geometrischen Daten der Stütze und der vorbekannten positionellen Zuordnung zwischen der Stütze und der Navigations-Referenzeinrichtung und aufgrund der Positionsdaten des Röntgen- bzw. Fluoroskopiegerätes die Abbildung der Stütze in einer Röntgen- bzw. Fluoroskopieaufnahme bei der Registrierung unberücksichtigt zu lassen.

Die Stütze besteht bevorzugt schon aus einem für Röntgenstrahlen durchlässigen Material, und bei einer Ausgestaltungsvariante ist/sind an der Stütze im bekannten Abstand zu den Stützenelementen ein Marker oder mehrere Marker aus einem für Röntgenstrahlung im Wesentlichen undurchlässigen Material angeordnet. Diese Marker dienen als Identifizierungshilfsmittel bzw. "Ausgangspunkte" für die Berechnung der Lage der Stützenabbildung.

Es besteht im Rahmen der Erfindung ferner die Möglichkeit, den Positionierer selbst so zu gestalten, dass er, obwohl er das Becken im Bereich zwischen den ASIS- und Pubis-Landmarken hält, das Abgreifen dieser Landmarken mit einem Navigations-Pointer gestattet, um so diese Punkte bei der Registrierung zu digitalisieren. Dies kann durch eine Formgebung für die Stütze geschehen, welche einen freien Zugang zu diesen Landmarkenpunkten gestattet.

Die Erfindung umfasst weiter ein Programm, das, wenn es auf einem Computer läuft oder in einem Computer geladen ist, den Computer veranlasst, eines der Verfahren durchzuführen, wie sie oben beschrieben worden sind. Ferner umfasst die Erfindung ein Computerprogramm-Speichermedium, das ein solches Programm aufweist.

Die Erfindung wird im Weiteren anhand der beiliegenden Zeichnungen näher erläutert. Sie kann alle hierin beschriebenen Merkmale einzeln sowie in jedweder Kombination umfassen. In den Zeichnungen zeigen:
- Figur 1: ein generelles Operationssaal-Setup für die Durchführung einer Beckenregistrierung mit Hilfe der vorliegenden Erfindung;
- Figur 2: eine Ansicht von oben auf einen abgestützten Patienten;
- Figur 3: eine perspektivische Ansicht einer Patienten-Stützvorrichtung mit Stütze und Stützenhalterung;
- Figur 4: ein Detail der Stützenhalterung aus Figur 3;
- Figur 5: eine schematische Ansicht der Patienten-Beckenabstützung; und
- Figur 6: eine Ausführungsform einer Stütze zur Verwendung mit der vorliegenden Erfindung.

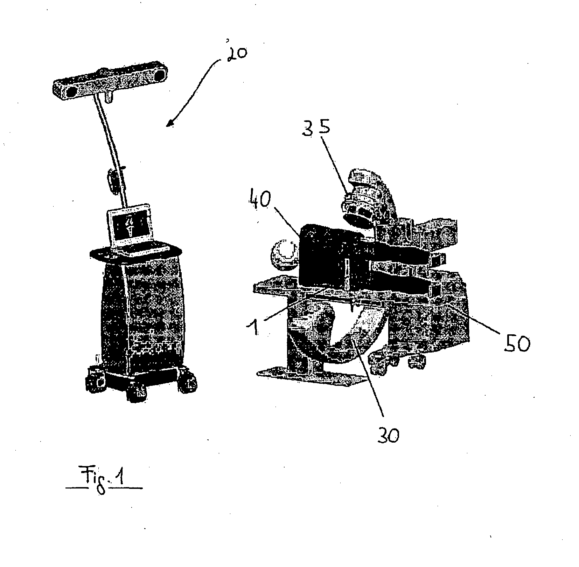

Einen allgemeinen Überblick über das Anwendungsumfeld der vorliegenden Erfindung gibt die Figur 1. Dort ist ein Patient 40 dargestellt, der seitlich auf der Patientenliege 50 liegt. Der Patient ist abgestützt bzw. fixiert, und die vordere Stütze des hierzu verwendeten Patientenpositionierers ist mit dem Bezugszeichen 1 angedeutet. Zur Erstellung von Röntgenbildern, insbesondere zur Fluoroskopie-Unterstützung der Beckenregistrierung ist an der Patientenliege 50 ein C-Bogen-Fluoroskop 30 angeordnet, das an seiner Strahlenquelle ein so genanntes Registrierungskit 35 aufweist. Mit Hilfe des Registrierungskits 35, das Referenzeinrichtungen aufweist, kann die Lage des C-Bogens unter Verwendung eines medizintechnischen Navigationssystems 20 ermittelt werden, das links von der Patientenliege steht. Somit kann auch die Lage der gemachten Fluoroskopieaufnahme im Raum bestimmt werden.

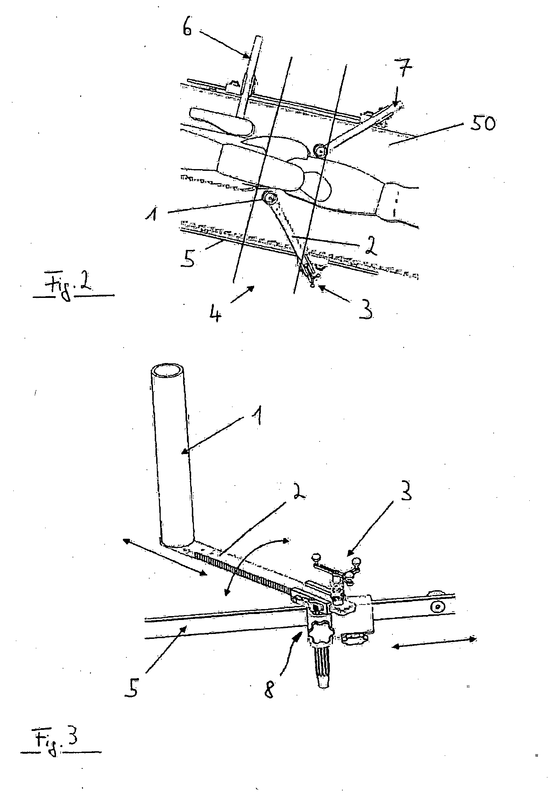

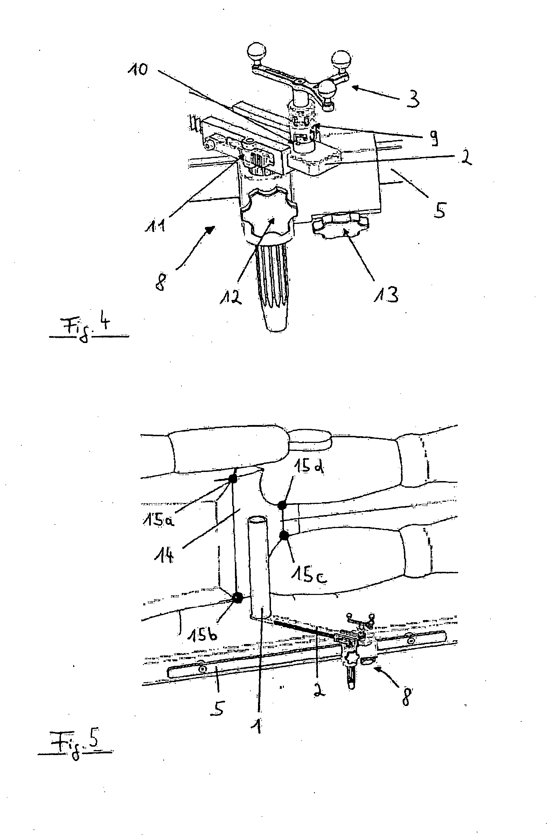

Auch die Haltevorrichtung für die Stütze 1 trägt eine Navigationsreferenz 3. Diese Navigationsreferenz ist in der Figur 1 wegen der Größenverhältnisse nicht sichtbar, sie ist aber in den Figuren 2 bis 5 dargestellt. Die Figur 2 zeigt in einer Ansicht von oben den auf der Patientenliege 50 liegenden Patienten, der mit drei Stützvorrichtungen im Beckenbereich abgestützt ist. Die beiden hinteren Stützvorrichtungen sind im Ganzen mit dem Bezugszeichen 6 und 7 bezeichnet worden, die vordere Stützeinrichtung ist detaillierter bezeichnet, und ihre Bestandteile sind auch in den Figuren 3 bis 5 zu sehen. Sie umfasst die stabartige Stütze 1 sowie den Haltearm 2, auf dem am anderen Ende eine Referenzeinrichtung 3 angebracht ist. Diese Referenzanordnung 3 weist drei Markerkugeln auf, die vom Navigationssystem 20 (Figur 1) erfasst werden können, und mit dieser Erfassung wird wegen der starren Positionszuordnung zwischen Stütze 1 und Referenzeinrichtung 3 die genaue Lage der Stütze 1 im Raum bekannt.

Aus der Figur 2 ist noch erkennbar, dass sich Teile der Stützvorrichtung, nämlich die Stütze 1 selbst sowie Teile des Arms 2 und der Haltevorrichtung 8 in dem Strahlengang des Fluoroskopiegeräts 30 vor dem Becken befinden, wobei der Strahlengang durch die zwei parallelen Linien mit dem Bezugszeichen 4 angedeutet ist.

Die Haltevorrichtung 8 ist an einer Schiene 5 verschieblich befestigt, die an der Patientenliege 50 fixiert ist. Mit dem Dreh-Schnellverschluss 13 kann die Position der Haltevorrichtung 8 entlang der Schiene 5 fixiert werden. Das Bezugszeichen 12 deutet auf einen Schnellverschluss-Mechanismus, der die Drehung bzw. das Verschwenken des Armes 2 fixiert bzw. freigibt und der Schnellverschluss-Mechanismus 11 gestattet es, den Arm in seiner Schiebe-Längsführung zu fixieren. Ein Adapter 10 für die Referenzeinrichtung ist fast am hinteren Ende des Armes 2 angeordnet, und auf diesem Adapter 10 ist ein Schnellverschluss-Mechanismus 9 für die Referenzeinrichtung 3 aufgesetzt, der das schnelle Anbringen und Abnehmen der Referenzeinrichtung 3 gestattet. Mit Hilfe dieser Konstruktion kann nunmehr die Stütze 1 an einem gewünschten Punkt vorne am Becken des Patienten positioniert werden, um diesen zu fixieren.

Die Ausgestaltung der Stütze 1 als länglicher Stab und die Bewegungsmöglichkeiten sowie Fixierungsmöglichkeiten der Halteeinrichtung 8 (Schwenkbewegung des Armes 2, Schiebebewegung des Armes 2 und seitliche Verstellung an der Schiene 5) gewährleisten eine sehr freie Positionierungsmöglichkeit für die Stütze 1, wobei gleichzeitig ein fester Halt in der Fixierungsposition gegeben ist. Hieraus ergibt sich nun die Möglichkeit, den Patienten schon an der Vorderseite des Beckens so zu fixieren, dass die ASIS- und Pubis-Punkte 15a, 15b, 15c, 15d nicht von der Stütze 1 abgedeckt werden, sondern die Stütze 1 zwischen diesen Punkten zu liegen kommt. Diese Körperlandmarken können dann bei fixiertem Patientenbecken mit einem Navigationspointer angefahren und ihre Lage registriert werden.

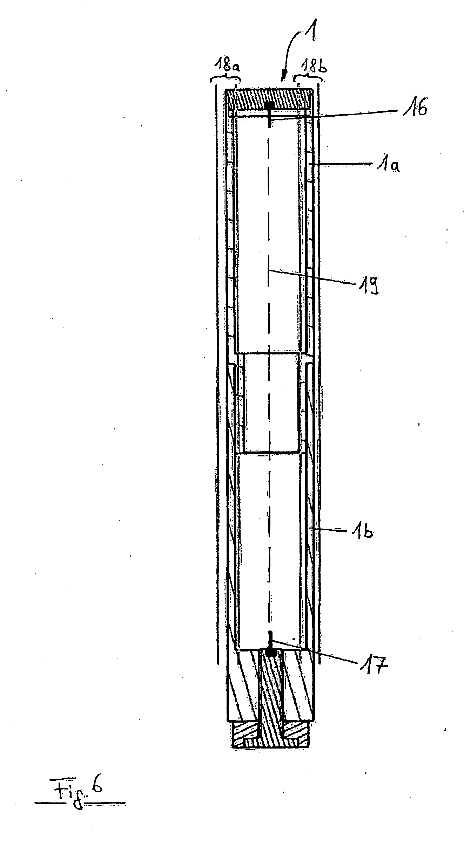

Neben der erfindungsgemäßen Verwendung einer Navigations-Referenzeinrichtung 3 eignet sich auch die in Figur 6 im Längsschnitt dargestellte Stütze 1 für die Artefakt-Eliminierung. Sie ist aus einem Oberteil 1a und einem Unterteil 1b aufgebaut, die in der Mitte zusammengesteckt sind. Sie ist röhrenförmig, und sie besteht im Wesentlichen aus einem für Röntgenstrahlung durchlässigen Material. Im Deckel und im unteren Einsatz der Stütze 1 sind Marker 16 und 17 eingesetzt, die aus einem für Röntgenstrahlung nicht durchlässigem Material bestehen, und mit den Bezugszeichen 18a und 18b sind Bereiche angedeutet, welche die Kantenkonturen der Stütze 1 umfassen. Eine gedachte Verbindungslinie zwischen den Markern 16 und 17 ist mit dem Bezugszeichen 19 bezeichnet.

Die Registrierung mit einer Stütze gemäß Figur 6 wird wie folgt durchgeführt: Ganz allgemein muss zur Navigationsunterstützung die Lage der vorderen Beckenebene mit dem Navigationssystem ermittelt werden, wobei diese Lage durch die ASIS- und Pubis-Punkte 15a bis 15d bestimmt wird. Bei einer fluoroskopischen Registrierung mit dem Fluoroskopiegerät 30 (Figur 1) werden hierzu am Becken und am Oberschenkel des Patienten Referenzanordnungen befestigt. Es werden zwei Fluoroskopbilder im Bereich des Beckens erstellt und der ASIS-Punkt auf der zu behandelnden Seite wird mit einem Navigationspointer angefahren und registriert. Das System errechnet dann die anatomischen Landmarken des Beckens speziell in der Anterior-Beckenebene und der mittleren Sagittalebene basierend auf den abgebildeten Kanten des Beckens auf der Fluoroskopieaufnahme.

Diese Kanten werden aber möglicherweise durch die Kanten bzw. Konturen der Stütze 1, die trotz der Strahlendurchlässigkeit als Artefakte mit abgebildet werden, gestört. Wenn das Fluoroskopiebild mit der Stütze 1 und den zusätzlichen Markern 16 und 17 im Strahlengang gemacht wird, bewirkt die Abbildung der Stütze, dass sich Schatten und Kanten auf der Abbildung befinden. Weil die Stütze aus einem anderen Material bzw. unterschiedlichen Materialien besteht, entstehen auf der Aufnahme unterschiedliche Grauwerte. Weil die Marker 16 und 17 aus einem für Röntgenstrahlen nicht durchlässigen Material (Stahl) hergestellt sind, bilden sie sich fast schwarz auf der Aufnahme ab. Die Kanten der Stütze erscheinen in einer leichten Graufärbung.

Durch eine erfindungsgemäß bereitgestellte Hard- und Software im Navigationssystem werden die schwarzen Abbildungen der Marker 16 und 17 auf der Aufnahme detektiert und es wird nach speziellen Grauwerten gesucht, die in einem vorbestimmten Abstand zu den Markern 16, 17 bzw. zu der gedachten Verbindungslinie 19 stehen. Diese speziellen Abstände werden beispielsweise durch die Bereiche 18a und 18b in Figur 6 aufgezeigt. Sie müssen die Kanten der Stützenabbildung umfassen. Diese Kanten mit ihren speziellen Grauwerten werden dann bei der abfolgenden Erfassung der anatomischen Strukturen des Beckens nicht mehr berücksichtigt, so dass sichergestellt wird, dass die für die Registrierung verwendeten Beckenkonturen auch tatsächlich abgebildete Beckenlinien beschreiben. Die Registrierung kann dann aufgrund der Artefakt-Eliminierung korrekt stattfinden und die von ihr abhängige Navigation des Eingriffs wird die positionsrichtige Unterstützung liefern.

## Patentansprüche

1. Artefakt-Eliminierungsverfahren für ein medizintechnisches Beckenregistrierungsverfahren zur positionellen Registrierung eines mit mindestens einer Stütze (1) abgestützten Patientenbeckens (14), mit den folgenden Schritten:
- geometrischen Daten der Stütze (1) und eine vorbekannte positionelle Zuordnung zwischen der Stütze (1) und einer Navigations-Referenzeinrichtung (3) werden im Datenspeicher eines für die Registrierung verwendeten medizintechnischen Navigationssystems (20) abgespeichert,
- mindestens zwei Röntgenaufnahmen des Patientenbeckens (14) werden erstellt, wobei die Stütze (1) sich im Strahlengang der Aufnahme befindet,
- die Position der Stütze (1) und eines Röntgengeräts (30) während einer Aufnahme werden durch das Navigationssystem (20) basierend auf der Erfassung der der Stütze positionell zugeordneten Navigations-Referenzeinrichtung (3) ermittelt,
- mit Hilfe der ermittelten Positionsdaten für die Stütze (1) und das Röntgengerät (30) wird die Abbildung der Stütze (1) in der Aufnahme identifiziert,
- die Aufnahme wird für die Registrierung verwendet, wobei die Abbildungselemente der identifizierten Abbildung der Stütze (1) als bei der Registrierung nicht zu verwendende Teile der Abbildung definiert werden.

2. Verfahren nach Anspruch 1, bei dem Abbildungselemente der identifizierten Abbildung der Stütze (1) von der Aufnahme subtrahiert werden.

3. Verfahren nach Anspruch 2, bei dem als Abbildungselemente abgebildete Konturen der Stütze (1) in der Aufnahme identifiziert und als nicht zu verwendende Teile definiert werden.

4. Verfahren nach einem der Ansprüche 1 bis 3, bei welchem bei der Identifizierung der Stützenabbildung zusätzlich die Grauwerte von Stützen-Abbildungselemente als Identifizierungshilfsmittel verwendet werden, insbesondere bekannte oder im Wesentlichen bekannten Grauwerte, wie sie von den Materialien der Stütze (1) üblicherweise erzeugt werden.

5. Verfahren nach einem der Ansprüche 1 bis 4, bei welchem bei der Identifizierung der Stützenabbildung zusätzlich bekannte Abstände von Stützenelementen zu deutlich abbildbaren Stützenelementen als Identifizierungshilfsmittel verwendet werden.

6. Verfahren nach einem der Ansprüche 1 bis 5, bei welchem bei der Identifizierung der Stützenabbildung bekannte Abstände von Stützenelementen zu speziellen, deutlich abbildbaren Marker an der Navigations-Referenzeinrichtung (3) als Identifizierungshilfsmittel verwendet werden.

7. Verfahren nach einem der Ansprüche 1 bis 6, bei welchem bei der Identifizierung der Stützenabbildung zusätzlich bekannte Abstände von Stützenelementen zu Verbindungslinien von speziellen, deutlich abbildbaren Markern in oder an der Stütze (1) als Identifizierungshilfsmittel verwendet werden.

8. Artefakt-Eliminierungssystem zur Eliminierung von Artefakten in Röntgenaufnahmen, die bei einem medizintechnischen Beckenregistrierungsverfahren verwendet werden, das zur positionellen Registrierung eines mit mindestens einer Stütze (1) abgestützten Patientenbeckens (14) dient, mit
a) einem Röntgengerät (30);
b) einem medizintechnischen Navigationssystem (20), das eingerichtet ist, Positionsdaten des Röntgengeräts zu bestimmen;
c) einer Trackingvorrichtung, welche die Position einer Navigations-Referenzeinrichtungen (3) bestimmen kann;
d) einer der Stütze (1) in vorbekannter Weise positionell zugeordneten Navigations-Referenzeinrichtung (3);
e) einem Datenspeicher im Navigationssystem (20), in dem die geometrischen Daten der Stütze (1) und die vorbekannte positionellen Zuordnung zwischen Stütze (1) und der Navigations-Referenzeinrichtung (3) abgespeichert werden; und
f) einer Logik im Navigationssystem (20), welche eingerichtet ist, aufgrund der geometrischen Daten der Stütze (1) und der vorbekannten positionellen Zuordnung zwischen der Stütze (1) und der Navigations-Referenzeinrichtung (3) und aufgrund der Positionsdaten des Röntgengerätes (30) die Abbildung der Stütze (1) in einer Röntgenaufnahme bei der Registrierung unberücksichtigt zu lassen.

9. Artefakt-Eliminierungssystem nach Anspruch 8, **dadurch gekennzeichnet, dass** die Stütze (1) aus einem für Röntgenstrahlung im Wesentlichen durchlässigen Material besteht.

10. Artefakt-Eliminierungssystem nach Anspruch 8 oder 9, **dadurch gekennzeichnet, dass** an der Stütze (1) in bekanntem Abstand zu den Stützenelementen ein Marker oder mehrere Marker aus einem für Röntgenstrahlung im Wesentlichen undurchlässigen Material an der Navigations-Referenzeinrichtung (3) angeordnet sind.

11. Programm, das, wenn es auf einem Computer läuft oder in einem Computer geladen ist, den Computer veranlasst, ein Verfahren gemäß einem der Ansprüche 1 bis 7 durchzuführen.

12. Computerprogramm-Speichermedium, das ein Programm nach Anspruch 11 aufweist.

## Claims

1. An artefact elimination method for a medical pelvic registration method for positionally registering a patient's pelvis (14) which is supported by at least one support (1), comprising the following steps:
- geometric data of the support (1) and a prior-known positional assignment between the support (1) and a navigation reference means (3) are stored in the data memory of a medical navigation system (20) which is used for registering;
- at least two x-ray recordings of the patient's pelvis (14) are produced, wherein the support (1) is situated in the radiation path of the recording;
- the position of the support (1) and the position of an x-ray apparatus (30) during a recording are ascertained by the navigation system (20) on the basis of detecting the navigation reference means (3) which is positionally assigned to the support;
- with the aid of the ascertained positional data for the support (1) and the x-ray apparatus (30), the image of the support (1) is identified in the recording;
- the recording is used for registering, wherein the image elements of the identified image of the support (1) are defined as parts of the image which are not to be used in registering.

2. The method according to Claim 1, wherein image elements of the identified image of the support (1) are subtracted from the recording.

3. The method according to Claim 2, wherein imaged contours of the support (1) are identified in the recording as image elements and defined as parts which are not to be used.

4. The method according to any one of Claims 1 to 3, wherein when identifying the support image, the grey values of support image elements are additionally used as identification aids, in particular known or substantially known grey values such as are usually generated by the materials of the support (1).

5. The method according to any one of Claims 1 to 4, wherein when identifying the support image, known distances between support elements and support elements which can be clearly imaged are additionally used as identification aids.

6. The method according to any one of Claims 1 to 5, wherein when identifying the support image, known distances between support elements and specific markers, which can be clearly imaged, on the navigation reference means (3) are used as identification aids.

7. The method according to any one of Claims 1 to 6, wherein when identifying the support image, known distances between support elements and connecting lines of specific markers, which can be clearly imaged, in or on the support (1) are additionally used as identification aids.

8. An artefact elimination system for eliminating artefacts in x-ray recordings which are used in a medical pelvic registration method which serves to positionally register a patient's pelvis (14) which is supported by at least one support (1), comprising:
- an x-ray apparatus (30);
- a medical navigation system (20) which is designed to determine positional data of the x-ray apparatus;
- a tracking device which can determine the position of a navigation reference means (3);
- a navigation reference means (3) which is positionally assigned to the support (1) in a prior-known way;
- a data memory in the navigation system (20), in which the geometric data of the support (1) and the prior-known positional assignment between the support (1) and the navigation reference means (3) are stored; and
- a logic in the navigation system (20) which is designed to disregard, on the basis of the geometric data of the support (1) and the prior-known positional assignment between the support (1) and the navigation reference means (3) and on the basis of the positional data of the x-ray apparatus (30), the image of the support (1) in an x-ray recording when registering.

9. The artefact elimination system according to Claim 8, **characterised in that** the support (1) consists of a material which is substantially permeable to x-ray radiation.

10. The artefact elimination system according to Claim 8 or 9, **characterised in that** one or more markers made of a material which is substantially impermeable to x-ray radiation is/are arranged on the navigation reference means (3) on the support (1) at a known distance from the support elements.

11. A program which, when it is running on a computer or is loaded on a computer, causes the computer to perform a method in accordance with any one of Claims 1 to 7.

12. A computer program storage medium which comprises a program according to Claim 11.

## Revendications

1. Procédé d'élimination d'artefacts pour un procédé médico-technique d'enregistrement d'un bassin pour l'enregistrement de la position d'un bassin (14) d'un patient supporté au moyen d'au moins un support (1) avec les étapes suivantes :
- enregistrement de données géométriques du support (1) et d'une correspondance déjà connue des positions du support (1) et d'un dispositif de référence pour la navigation (3) dans la mémoire d'un système de navigation (20) médico-technique utilisé pour l'enregistrement,
- réalisation d'au moins deux radiographies du bassin du patient (14), où le support (1) se trouve sur la trajectoire des rayons lors de la réalisation du cliché,
- la position du support (1) et d'un appareil radiographique (30) lors de la réalisation d'un cliché est déterminée par le système de navigation (20) à partir de la détection du dispositif de référence pour la navigation (3) attribué en termes de position au support,
- l'image du support (1) dans le cliché est identifiée à l'aide des données de position déterminées pour le support (1) et pour l'appareil radiographique (30),
- le cliché est utilisé pour l'enregistrement, où les éléments d'image de l'image du support (1) identifiée sont définis comme des parties de l'image ne devant pas être utilisées lors de l'enregistrement.

2. Procédé selon la revendication 1, lors duquel les éléments d'image de l'image du support (1) identifiée sont soustraits du cliché.

3. Procédé selon la revendication 2, lors duquel des contours du support (1) représentés comme éléments d'image dans le cliché sont identifiés et définis comme parties à ne pas utiliser.

4. Procédé selon l'une des revendications 1 à 3, lors duquel les niveaux de gris d'éléments d'image du support sont en outre utilisés comme aides à l'identification lors de l'identification de l'image du support, et notamment des niveaux de gris connus ou substantiellement connus comme étant habituellement générés par les matériaux du support (1).

5. Procédé selon l'une des revendications 1 à 4, lors duquel des distances déjà connues entre des éléments de support et des éléments de support susceptibles d'être représentés de manière nette sont en outre utilisées comme aides à l'identification lors de l'identification de l'image du support.

6. Procédé selon l'une des revendications 1 à 5, lors duquel des distances déjà connues entre des éléments de support et des marqueurs spéciaux sur le dispositif de référence pour la navigation (3) susceptibles d'être représentés de manière nette sont utilisés comme aides à l'identification lors de l'identification de l'image du support.

7. Procédé selon l'une des revendications 1 à 6, lors duquel des distances déjà connues entre des éléments de support et des lignes reliant des marqueurs spéciaux dans ou sur le support (1) susceptibles d'être représentés de manière nette sont en outre utilisées comme aides à l'identification lors de l'identification de l'image du support.

8. Système d'élimination d'artefacts pour l'élimination d'artefacts dans des clichés radiographiques utilisés lors d'un procédé médico-technique d'enregistrement d'un bassin servant à l'enregistrement de la position d'un bassin (14) d'un patient supporté au moyen d'au moins un support (1), avec
a) un appareil radiographique (30) ;
b) un système de navigation médico-technique (20) configuré pour déterminer des données de position de l'appareil radiographique ;
c) un dispositif de suivi pouvant déterminer la position d'un dispositif de référence pour la navigation (3) ;
d) un dispositif de référence pour la navigation (3) attribué en termes de position au support (1) de manière connue ;
e) une mémoire de stockage de données dans le système de navigation (20), dans lequel sont enregistrées les données géométriques du support (1) et la correspondance déjà connue entre les positions du support (1) et du dispositif de référence pour la navigation (3); et
f) une logique dans le système de navigation (20) configurée pour ne pas prendre en compte, lors de l'enregistrement, l'image du support (1) dans un cliché radiographique, déterminée à partir des données géométriques du support (1) et de la correspondance déjà connue entre les positions du support (1) et du dispositif de référence pour la navigation (3) ainsi qu'à partir des données de position de l'appareil radiographique (30).

9. Système d'élimination d'artefacts selon la revendication 8, **caractérisé en ce que** le support (1) est composé d'un matériau substantiellement perméable aux rayons X.

10. Système d'élimination d'artefacts selon la revendication 8 ou 9, **caractérisé en ce que** un ou plusieurs marqueurs composés d'un matériau substantiellement perméable aux rayons X sont disposés sur le dispositif de référence pour la navigation (3) sur le support (1) à une distance connue des éléments de support.

11. Programme qui, lorsqu'il est exécuté ou chargé dans un ordinateur, provoque l'exécution par l'ordinateur d'un procédé selon l'une des revendications 1 à 7.

12. Support de données pour un programme d'ordinateur, comportant un programme selon la revendication 11.
